# EUROPEAN PATENT APPLICATION

(11) **EP 1 988 096 A1**
(43) Date of publication of application: **05.11.2008**
(21) Application number: 07107041.1
(22) Date of filing: 26.04.2007
(51) Int. Cl.: C07H 3/06, C08B 37/00, A23L 1/29

(54) **Process for preparing a galactooligosaccharides powder**

(71) Applicant: LABORATORIOS ORDESA, S.L, 08830 Sant Boi de Llobregat, Barcelona (ES)
(72) Inventor: Salvadó Solervicens, Ramón, 08830 Sant Boi de Llobregat (Barcelona) (ES); Chifré Petit, Ricard, 08830 Sant Boi de Llobregat (Barcelona) (ES); Santpere Gibert, Josep, 08830 Sant Boi de Llobregat (Barcelona) (ES); Cuartero Olona, Fernando, 08830 Sant Boi de Llobregat (Barcelona) (ES); Martín Palomas, Manel, 08830 Sant Boi de Llobregat (Barcelona) (ES)
(74) Representative: Barlocci, Anna

(57) **Abstract**

The invention provides a process for the preparation of a galactooligosaccharides (GOS) product with a content of water equal or lower than 2% by weight, comprising the following steps: (i) mixing at least water, GOS-containing syrup and a carrier, in continuous agitation; (ii) drum drying the mixture of step (i) in a drum dryer; and (iii) sieving the product resulting from step (ii). After this, the product can be milled to obtain a GOS powder. The invention also provides a GOS powdered product of high purity obtainable by said process, food products and nutritional compositions comprising it, and its use as a prebiotic.

## Description

The present invention relates to a process for the preparation of a galactooligosaccharides powder, compositions comprising it and the use thereof as ingredient to be included in food products.

### BACKGROUND ART

The aim of human nutrition is to provide a suitable contribution of energy and nutrients. The so-called "functional foods" are functional components that are added to provide beneficial effects in human specific functions, giving positive results for well-being and health and reducing the risk to diseases. Within functional foods there is currently a great interest in the role of prebiotics as promoters of bifidogenic flora in human, since they provide nutrients for all colonic bacteria, therefore contributing to a beneficial increase in the pH and bacterial flora. Prebiotics are non-digestible food ingredients, essentially carbohydrates, that beneficially affect the host by selectively stimulating the growth and/or activity of one or a limited number of bacteria in the colon, thereby resulting in an improvement in the health of the host. Furthermore, prebiotics have an important role in immune gastrointestinal functions, in mineral bioavailability, in the correct lipid metabolism and in the prevention of colon carcinogenesis.

Within prebiotics, galactooligosaccharides, known as GOS, are generally represented by the formula Gal-(Gal)n-Glc, wherein Gal represents a galactose residue, Glc represents a glucose residue and n represents an integer representing the number of galactose units linked to the terminal glucose unit. The number of saccharide units (Gal and Glc units) in one molecule, i.e. the values n+2 in the above formula, is commonly referred to as degree of polymerization (DP).

GOS are obtained from lactose and they have a great similarity to the ones from breast milk, so they are very used in infant nutrition. Many studies demonstrate that dairy formulas with supplements of GOS and other oligosaccharides, stimulate beneficial intestinal flora, reduce the non-desired flora, contribute in the prevention of allergic pathologies and metabolic alterations, collaborate in immune functions and optimize physiological processes not only in infants but also in adult humans.

GOS can be produced from lactose by the transglycosylating activity of beta-galactosidases. Such a method generally produces a sugar mixture containing about 30 to 70% of GOS, disaccharides mainly comprising unreacted lactose and monosaccharides such as glucose, galactose and other secondary products. The resulting GOS preparations are in the form of solutions or thick syrups of low purity in GOS and not easy to treat because of their liquid state. GOS are difficult to handle, i.e. they are produced in aqueous solutions with low dry matter content, and hence prone to bacterial contamination and thus difficult to store. Additionally, GOS are prone to maillardation, particularly when brought into contact with other carbohydrates and protein, potentially resulting in undesirable Maillard reaction products with concomitant undesirable effects on color, odor and taste of the product.

EP 435657 attempts to overcome these disadvantages with a modified process to obtain GOS from lactose. The process comprises heating lactose or a mixture of lactose and galactose in the presence of an inorganic acid as a catalyst in an anhydrous powder condition. The lactose is an alpha-lactose, beta-lactose or equilibrated lactose. The inorganic acid is hydrochloric acid. The lactose is heated to 100-200 °C. The GOS are prepared in high purity, and being in powder form are easy to treat.

Many times food industry needs raw materials in powdered form to be included in dried commercial nutritional formulations or in dried mixtures for internal processes. In such a case, at present, GOS preparations are spray-dried to result in dryer GOS preparations.

L. Curda et al., "Dried buttermilk containing galactooligosaccharides -process layout and its verification", Journal of Food Engineering, 2006, vol. 77, pp. 468-471, relates to a dried buttermilk with a concentration of GOS of 70 g/kg.

JP 04030773 relates to powders manufactured by dissolving water-soluble dietary fibers (guar gum) with oligosaccharide-containing syrups and spray-drying. A syrup [solid content 70%, GOS content (based on the solid) 50%] 114.3, the guar gum hydrolyzate solution 66.7, and H₂O 105 weight parts are mixed, and spray-dried to manufacture a powder which do not show aggregation nor liquefaction even after kept at 20 °C and relative humidity 60% for 72 h.

EP 458358 relates to a skim-milk powder rich in GOS prepared by treatment of a concentrated skim milk with β-galactosidase and the enzyme is denatured by heating and the product spray-dried to a powder. After heating and spray-drying, a skim milk powder 3500 kg containing 10.5 weight % GOS is recovered.

JP 05059091 relates to pharmaceuticals containing GOS and lactose as active ingredients. Lactose is treated with β-galactosidase in H₂O at 40 °C and pH 4.5 for 10 h to produce powder containing ≥ 95 weight % GOS.

Due to the chemical nature of GOS, many of the dried GOS preparations still contain a high percentage of water. Therefore the resulting GOS preparations have the same handling problems indicated above. In fact there is not any stable powder GOS product in the market with the technical specifications to be used as a stable dried ingredient.

Because of the presumed difficulties attached to their use, the GOS are not or only very limitedly used in food applications. Thus, there is a need for new processes to provide stable powdered GOS preparations.

### SUMMARY OF THE INVENTION

The object of the present invention is to provide a powder of galactooligosaccharides of high purity to be used in food industry through a simple method.

Accordingly, the present invention refers to a process for the preparation of a galactooligosaccharides product with a content of water equal or lower than 2% by weight, comprising the following steps: (i) mixing at least water, galactooligosaccharides-containing syrup and a carrier, in continuous agitation; (ii) drum drying the mixture of step (i) in a drum dryer; and (iii) sieving the product resulting from step (ii).

The present inventors have found that drum drying aqueous products comprising galactooligosaccharides, yields highly beneficial powders, which can be suitably used for food applications. Thus, the present invention also provides a galactooligosaccharides product obtainable by the process described herein, food products and nutritional compositions comprising it, and its use as a prebiotic.

These and other objects of the present invention will be further described in the detailed description section that follows.

### DETAILED DESCRIPTION OF THE INVENTION

In general, an oligosaccharide that contains galactose in its molecule is called a galactooligosaccharide, however, in the present invention, the term "galactooligosaccharide" means a saccharide which contains from 2 to 10 saccharide units hereinafter. Galactooligosaccharides is referred as "GOS" in this description.

### GOS sources

Raw GOS to be used through the process of the invention can be produced from lactose by different methods known in the art. Essentially, GOS are obtained from lactose by the transglycosylating activity of beta-galactosidase derived from e.g. *Aspergillus oryzae* or *Cryptococcus.* Betagalactooligosaccharides consist of a number of beta-1,6-linked galactosyl residues linked to a terminal glucose unit via a beta-1,4-bond. Another type of GOS is isolated from soybeans. These alpha-galactooligosaccharides (galactosyl-sucrose oligosaccharides) include raffinose, stachyose and verbascose and consist of galactose residues linked alpha-1,6 to the glucose moiety of sucrose. The physiological effects of these oligosaccharides appear to be similar to the beta-linked galactose oligomers. The composition of GOS may vary in chain length and in type of linkage between the monomer units.

First, as for lactose to be used as a material, there is no restriction in its kind, thus all kinds of conventionally known lactose being widely employed. For example, lactose commercially available on the market can be employed, and each of alpha-lactose, beta-lactose and equilibrated lactose can be used in case of adding acid in a solution of lactose. In case of adding acid into powder of lactose, alpha-lactose is recommendable. Alpha-lactose is the most common kind of lactose obtained by separating crystals out of a solution containing lactose crystallized at a relatively low temperature (usually below 94 DEG C). Beta-lactose is a kind of lactose obtained by separating crystals out of a solution containing lactose crystallized at a high temperature (usually above 94 DEG C). Equilibrated lactose is a kind of lactose obtained by spray-drying a solution containing lactose and is an amorphous powder containing alpha-lactose and beta-lactose in an approximate ratio of 4:6. When dissolved in water, each of the mentioned lactoses presents the same containing ratio of alpha-lactose and beta-lactose just as that in equilibrated lactose with the lapse of time. Secondly, galactose of an article commercially available on the market can be employed.

A suitable GOS syrup as raw material for the purpose of the invention is one which contain GOS and remains of lactose, glucose, galactose and water. In particular, the GOS syrup contains 44.25 g of GOS, 15.75 g of lactose, 14.25 g of glucose, 0.75 g of galactose, in a 75% of the syrup total weight (25% of water).

### Carriers

The function of the carriers in this invention is to allow to dry the GOS product at high temperature. It is desired to achieve the maximum concentration of pure GOS with the minimum concentration of carrier. Suitable carriers for the purposes of this invention are those with a composition based on carbohydrates, preferably rich in polysaccharides. Preferred carriers are those from vegetal flours, previously hydrolyzed or not: wheat flour, oat flour, rice flour, corn flour, barley flow, sorghum flour, rye flour, millet flour, carrot flour, potatoe flour, soy, legumes, and maltodextrins. Other suitable carriers for this invention are those with a composition based on proteins, such as whey milk protein and skimmed milk. These raw materials have a water content within the permitted ranges of specifications.

### Preparation process

### (i) Mixture

Preestablished amounts according to formula, of at least water, GOS syrup, and carrier, are poured into a working capacity stainless steel tank with agitator. In a particular embodiment of the invention, the mixture is formed by water, GOS syrup and the carrier. It is shacked during a preestablished time in order to achieve a homogeneous mixture, maintaining agitation until mixture unloading. The result is an aqueous dispersion with high concentration in solids.

In a particular embodiment of the invention, the GOS syrup is in an amount between 25 and 80%, and preferably in an amount between 50 and 60% of the total mixture weight before adding the water.

In another particular embodiment, the water is added firstly, the carrier secondly and the GOS-containing syrup finally, with continuous agitation.

### Dextrination (optional)

Before the previous mixture, flour and water in preestablished amounts are added to a covered reactor. A process of dextrination is carried by means of two phases of hydrolysis, and regulating temperature, time and pH with alkali or acid. The result is a concentrated viscous fluid with a high concentration of solids which is stored and is dosed in the mixture of section (i).

### (ii) Drum drying

The present invention provides a process for the production of a powdered product from an aqueous solution as described hereinabove, comprising a drum drying step wherein the aqueous composition is drum dried to form a powder. The principle of the drum drying process (or roller drying) is that a thin film of material is applied to the smooth surface of a continuously rotating steam heated metal drum. The film of the dried material is continuously scrapped off by a stationary knife located opposite the point of application of the liquid material. The dryer consists of a single drum or a pair of drums with or without satellite rollers. The applied steam pressure in the drums can vary from 5 to 9 bar, depending on the product.

A suitable drum dryer consists of a smelting central cylinder (3 m table x 1.5 m diameter) with wall thickness from 28 to 35 mm, prepared to support inner pressure up to 12 bar. Overheated steam is introduced by the front part by means of Johnson system, which provides a controlled temperature at the surface of the cylinder allowing to evacuate condensed products. In its upper part there are five satellite cylinders with smaller diameter, four with movement in opposite direction than the central one and one in the same direction. In the middle it has a longitudinal blade that removes the dried product from the central cylinder. In order to dispense the product on surface, it has a system of adjustable sliding faucets on the table. In order to evacuate the dispersing steam (water), the machine is covered by a vapor stove extractor hood.

The homogeneous mixture is transported from the mixture tank through the pump of viscous substances to a lung deposit, and through the pump, it is transported to the upper part of the drying machines. The mixture is dispersed between the satellite cylinders so that these put a fluid layer of variable thickness on the surface of the central cylinder. The drying is regulated by means of regulating temperature (by means of regulating pressure) and regulating number of turns. Thus, in one embodiment of the invention, the central cylinder of the drum dryer is at a temperature between 140 and 200 °C, and preferably between 165 and 175 °C. According to this, the inner pressure of the central cylinder is from 5 to 9 bar, and preferably 7.5 bar. In another embodiment, the central cylinder of the drum dryer rotates at a speed between 2 and 6 rpm, and preferably, between 4 and 5 rpm. The satellite cylinders provide several layers of humid product to the central cylinder. The layer, during the complete turn of the central cylinder, evacuates the dispersing liquid (water) in vapor form and undergoes a drying process adhered to the cylinder. When the turn is completed, a pressure blade uniformly scrapes all over the table removing the released layer in veil and dropping it to the endless conveyor belt.

### (iii) Sieving and transport to silos

The veil is cut up and mechanically transported (conveyor belts) until a centrifugal siever, where it is sieved and it is homogenized in size, becoming reduced flakes of up to 10 mm of diameter and preferably, from 2 to 6 mm. These flakes can already be used for industrial food applications. By means of vacuum and sterile air impulsion, the flakes are pneumatically transported until big stainless steel silos in sterile conditions. The product is stored in the silos until it is unloaded to transform it into finished product.

It could be necessary by technical or commercial reasons to add a final minority ingredient to the stored product. To this end, a controlled weight of product is unloaded from the silo to a stainless steel mixer, and a known weight of the minority product is added on it. It is mixed and it is carried to the centrifugal siever, where it is optionally sieved again becoming small particle of 0.5 a 1.5 mm of diameter It is pneumatically transported towards a selected packing silo.

### (iv) Milling

In a particular embodiment, the process also comprises a step of milling the product resulting from step (iii) to obtain a GOS powder. From the packing silo the product in flakes is given to a hammers mill to obtain a uniform powder of 100-250 µm of diameter. This powder is transported until a milling silo with vent filters box.

All the metallic surfaces in contact with the product are stainless steel except the central cylinder. All the surfaces in contact with the product are cleaned after working via humidity (chlorinated alkali, chlorinated foaming detergent, rinsing until clean waters of washing) and disinfected (quaternary ammonium or oxygen peroxide in peracetic acid and rinsing until clean waters of washing). All the rooms and their surfaces are washed and disinfected in similar conditions.

### Packing

The powder product, gathered in the milling silo, is given mechanically to the feeding hopper of a vertical packing machine with volumetric dosing by conveyor belt. The product is dosed, packed in flexible packages and adequately sealed.

An essential condition for obtaining of the product object of the present invention, is assuring the maximum asepsis. Also, it is necessary to have the suitable facilities in order to produce the product for the same process, whether containing habitual cereal flours or specific cereal flours to people with intolerance to habitual flours (e.g. flours with or without gluten). Total process time depends on the amount of material to be processed. Using a drum dryer of central cylinder of 3 m table x 1.5 m diameter, a usual yield is 300 kg of dried material obtained per hour.

### The GOS product

The present invention provides a GOS powdered product which comprises an amount of pure GOS up to 45% of the total dry weight. In a particular embodiment, the GOS product comprises an amount of pure GOS between 30 and 35% of the total dry weight. The product has a water content equal or below 2 wt.% based on the total weight of the product. This water content ensures an acceptable shelf life. Initial GOS DP does not change after the process. The GOS product of the invention can be in the form of flakes or powder according to the process described above. Both forms are useful for industrial food applications and the choice will depend on the final food product and the industrial process to obtain it.

In particular, the GOS powder obtained by the process of the invention has 20.25-20.88% of DP2 GOS (two monomers of Gal), 30.57-30.69% of DP3 GOS within the final composition. The analysis of the Gal profile is done following the AOAC Official Method 2001.1. "Determination of Trans-galactooligosaccharides in Selected Food Products by Ion-Exchange Chromatography".

The obtained GOS product has a little "water activity" and very low residual humidity. Still warm, it is transported with sterile and dry air to great silos, where it is stored under high hygienic conditions. When it is going to be sieved and transported with sterile and dry air towards a deposit for the packing, the operation takes place in a controlled environment zone with impulsion of filtered air and with a relative humidity degree of 50% maximum. In the packing operations zone (with previous milling or not), the relative humidity is also kept at 55% maximum, until the packing is sealed. The dried product is the result of a formulation designed to obtain a high concentration of dry GOS product, by itself an easily wettable substance. In addition, starting with an insoluble product, an intimate mixture of molecules of GOS protected (and its polarity) with hardly wettable molecules, gives a texture of very low hygroscopic capacity.

This product has very favorable rheological properties. It moves easily on stainless steel or plastic surfaces without agglomeration, compaction or granulation. It flows easily with gravity or by pneumatic impulse and facilitates enormously its metering in mixer dispensers, sieves, containers, etc. Since it has low hygroscopy, being in form of flakes or in form of powder of selectable particle size, it mixes easily and homogenously with other solid products. It also make easier its dispersion and dissolution in liquids. Since it does not absorb environmental humidity and has low residual humidity, the GOS product has a low risk of microbiological contamination and low risk of catalysis of degradation reactions of biomolecules. The dry product by its high concentration in GOS can be used in food industry. The weight to dose in industrial manufactures as in industrial bakery, cakes and pastries is minimum, corresponding to the nutritional amounts needed in a daily ingestion. The GOS product is advantageous in industrial manipulation because it is easy to dispense, comfortable to weigh, to dose, to mix, etc. It does not require special necessities of environmental humidity protection. It does not have problems neither in opening, closing, or transfer of packages, nor problems of agglomeration, granules, compaction or dampening.

### Industrial applications of the GOS product

The inventors have succeeded in preparing finely divided GOS powder which can be advantageously used in industry. The powder can be stored for a long time and can be used in the manufacture of food products.

The GOS product according to the invention is particularly suitable for use in human food. Therefore, the GOS product can be mixed with other ingredients in solid form to constitute a dry food. However the GOS powder can be used in the manufacture of non-dry final products, such as foams and emulsions. Other examples are table and butter spreads (oil-continuous or water continuous), cheeses (e.g. quark, cream, processed, soft and hard) and imitated cheeses, meat products (e.g. liver paste, frankfurter and salami sausages or meat spreads), chocolate spreads, fillings (e.g. truffle, cream) and frostings, chocolate, confectionery (e.g. caramel, fondants or toffee), dairy products (fermented milk, dairy drinks or desserts), frozen desserts (ice cream, sherbets,), baked goods (cakes, pastries), sauces and soups, coffee whiteners and so on. The GOS product according to the invention is also suitable for the manufacture of fodders for animal nutrition.

In another embodiment, the GOS powder of the invention is used for the manufacture of a nutritional composition. Examples are dairy products, functional foods, or food substitutes. Nutritional compositions also include dietary supplements, which intend to supply nutrients, (vitamins, minerals, fatty acids or amino acids) that are missing or not consumed in sufficient quantity in a person's diet (infants, pregnant women, elderly people, etc). In a particular embodiment, the GOS product is easily to homogenize with other ingredients, such as cereals or powdered milk to constitute an infant formula.

Unless otherwise defined, all technical and scientific terms used herein have the same meaning as commonly understood by a person skilled in the art to which this invention belongs. Methods and materials similar or equivalent to those described herein can be used in the practice of the present invention. Throughout the description and claims the word "comprise" and variations of the word, such as "comprising", are not intended to exclude other technical features, additives, components, or steps. Additional objects, advantages and features of the invention will become apparent to those skilled in the art upon examination of the description or may be learned by practice of the invention. The following examples and drawings are provided by way of illustration, and is not intended to be limiting of the present invention.

### DETAILED DESCRIPTION OF PARTICULAR EMBODIMENTS

Two reliability industrial tests for obtaining GOS powdered product were carried out to be reproduced in productive scale.

### EXAMPLE 1: Obtaining of dextrinated GOS powder with 18% of purity

In a mixer tank (capacity of 1500 I), 100 I of sanitary water were added. An operation of enzymatic dextrination of rice flour was conducted previously, obtaining a concentrated dextrin fluid. 80 kg of these fluid dextrins were weighed in an offset deposit and then added by means of a pump and hygienic transport to the mixer tank, shaking continuously with a double blade agitator at 142 rpm (5 min). 260 kg of rice flour were weighed in a solid weigher deposit and then added slowly to the mixer tank, until total interposition of the powder in the mixture of water and dextrins, maintaining permanent agitation (12 min). From an offset fluids weigher deposit, 150 kg of GOS syrup were added to the mixer tank until homogenization maintaining constant agitation (14 min). The homogeneous dispersion was transported by a small spindle pump until the lung deposit. From this, by means of a small spindle pump, with frequency changer, the fluid was hygienically transported to the meter and finally to the drum-dryer. The central roller was at 3.5 rpm and 6.5 bar. The fluid fills the baths between the roller satellites, beginning by the most distant one of the blade, and finishing with the bath of the motorized roller. When all the rollers were loaded of fluid and the impregnation of the central roller was total and homogenous, the lamina uprooted by the blade was observed. It was clean and dry. Then the central roller was set at 4.5 rpm and 7.5 bar. The obtained dry film was divided, sifted by 5 mm light, and milled to 200 µm of particle size. Samples were taken and analyzed. The resulting product was a dry, non unctuous, of good rheology, non hygroscopic palpable fine powder. It was white, creamy, with toasted cereal scent and sweet flavor. The residual humidity was 1.9%.

### EXAMPLE 2: Obtaining of dextrinated GOS powder with 30% of purity

In this case the operation of enzymatic dextrination of flour was not conducted.

In a mixer tank (capacity of 1500 I), 100 I of sanitary water were added. 150 kg of rice flour were weighed in a solid weigher deposit shaking continuously with a double blade agitator at 142 rpm (5 min). The flour was added slowly to the mixer tank, until total interposition of the powder in the water, having verified the correct dampening of all the powder, without formation of agglomerates (15 min). From the offset fluids weigher deposit, 255 kg of GOS syrup were added to the mixer tank with slow and constant pouring and permanent agitation until homogenization (12 minutes). 10 I of volumetrically measured sanitary water were added. From the offset solid weigher deposit, 50 kg of rice flour were added slowly to the mixer tank with constant agitation until homogeneity (5 min). The homogeneous dispersion (without agglomerates and all the solids finely damp) was transported by a small spindle pump until the lung deposit. From this, by means of a small spindle pump, with frequency changer, the fluid was hygienically transported to the meter and to the drum-dryer. The central roller was at 3 rpm and 6 bar. The fluid fills the baths between the roller satellites, beginning by the most distant one of the blade, and finishing with the bath of the motorized roller (10 min). When all the rollers were loaded of fluid and the impregnation of the central roller was total and homogenous, the lamina uprooted by the blade was observed. It was clean and dry. Then the central was set at 4.6 rpm and 7.5 bar. The obtained dry film was divided, sifted by 5 mm light, and milled to 200 µm of particle size. Samples were taken and analyzed. The resulting product was a dry, non unctuous, non forming agglomerates of good rheology, non hygroscopic palpable fine powder. It was white, creamy, with toasted cereal scent and sweet flavor. The residual humidity was 1.8%.

### EXAMPLE 3: Preparation of a cereal-based food for infants

A cereal-based food for infants in powder form was prepared using as ingredients, dextrinated cereals flour (wheat, oat, rice, corn, barley, sorghum, rye and millet), sugar, the GOS powdered product, malt extract, mineral salts, vitamins and aroma. The final formula is the following:

| | UNITS | NUTRITIONAL COMPOSITION per 100 g |
|---|---|---|
| Energy value | Kcal | 367 (1556 kJ) |
| Protein | g | 8.0 |
| Fat | g | 1.3 |
| Carbohydrates | g | 80.7 |
| Nutritional fiber | g | 5.5 |
| GOS | g | 3.0 (from 5.5 g Nutr. Fiber) |
| MINERALS: | g | 2.0 |
| Sodium | mg | 20 |
| Calcium | mg | 400 |
| Iron | mg | 8 |
| VITAMINS: | | |
| Vitamin A | µg | 450 (1500 IU) |
| Vitamin D | µg | 7.5 (300 IU) |
| Vitamin E | mg | 4.4 |
| Vitamin K | µg | 40 |
| Vitamin B₁ | mg | 0.5 |
| Vitamin B₂ | mg | 0.6 |
| Vitamin B₆ | mg | 0.8 |
| Vitamin B₁₂ | µg | 2 |
| Vitamin C | mg | 50 |
| Folic Acid | µg | 40 |
| Panthothenic acid | mg | 2.8 |
| Niacin | mg | 6 |
| Biotin | µg | 15 |

## Claims

1. A process for the preparation of a galactooligosaccharides product with a content of water equal or lower than 2% by weight, comprising the following steps:
(i) mixing at least water, galactooligosaccharides-containing syrup and a carrier, in continuous agitation;
(ii) drum drying the mixture of step (i) in a drum dryer; and
(iii) sieving the product resulting from step (ii).

2. The process according to claim 1, which further comprises the step of:
(iv) milling the product resulting from step (iii) to obtain a galactooligosaccharides powder.

3. The process according to any of the claims 1-2, wherein in step (i), the galactooligosaccharides syrup is in an amount between 25 and 80% of the total mixture weight before adding the water.

4. The process according to claim 3, wherein the galactooligosaccharides syrup is in a amount between 50 and 60% of the total mixture weight before adding the water.

5. The process according to claim 1, wherein the central cylinder of the drum dryer is at a temperature between 140 and 200 °C.

6. The process according to claim 5, wherein the temperature is between 165 and 175 °C.

7. The process according to claim 1, wherein the central cylinder of the drum dryer rotates at a speed between 2 and 6 rpm.

8. The process according to claim 7, wherein the speed is between 4 and 5 rpm.

9. The process according to claim 1, wherein the carrier is one that has a composition based on carbohydrates and it is rich in polysaccharides.

10. The process according to claim 9, wherein the carrier is selected from the group consisting of wheat flour, oat flour, rice flour, corn flour, barley flow, sorghum flour, rye flour, millet flour, carrot flour, potatoe flour, soy, legumes, and maltodextrins.

11. The process according to claim 1, wherein the carrier is one that has a composition based on proteins.

12. The process according to claim 11, wherein the carrier is selected from the group consisting of whey milk protein and skimmed milk.

13. A galactooligosaccharides product obtainable by the process defined in any of the claims 1-12.

14. The galactooligosaccharides product according to claim 13, which comprises an amount of pure galactooligosaccharides up to 45% of the total dry weight.

15. The galactooligosaccharides product according to claim 14, which comprises an amount of pure galactooligosaccharides between 30 and 35% of the total dry weight.

16. The galactooligosaccharides product according to any of the claims 13-15, wherein the particle diameter is lower than 10 mm.

17. The galactooligosaccharides product according to claim 16, wherein the diameter is between 100 and 250 µm.

18. A food product comprising the galactooligosaccharides product as defined in any of the claims 13-17.

19. The food product according to claim 18, which is selected from the group consisting of emulsions, foams, confectionery, fillings, chocolate, table and butter spreads, cheeses, meats products, dairy products, bakery goods, desserts, sauces, soups and fodders for animal food.

20. A nutritional composition comprising the galactooligosaccharides product as defined in any of the claims 13-17.

21. The nutritional composition according to claim 20, which is an infant formula.

22. Use of the galactooligosaccharides product as defined in any of the claims 13-17, as a prebiotic.
